# EUROPEAN PATENT SPECIFICATION

(11) **EP 2 508 707 B1**
(45) Date of publication and mention of the grant of the patent: **30.10.2019**
(21) Application number: 11161211.5
(22) Date of filing: 05.04.2011
(51) Int. Cl.: E21B 43/12, E21B 47/10

(54) **Monitoring the phase composition of production fluid from a hydrocarbon extraction well**
Überwachung der Phasenzusammensetzung von Produktionsflüssigkeit aus einem Kohlenwasserstoffextraktionsbohrloch
Surveillance de la composition de phase de fluide de production à partir d'un puits d'extraction d'hydrocarbure

(43) Date of publication of application: 10.10.2012
(73) Proprietor: GE Oil & Gas UK Limited, Nailsea Bristol BS48 1BS (GB)
(72) Inventor: Wingate, John Maclean, Bristol, BS7 8ER (GB)
(74) Representative: Stratagem IPM Limited

(56) References cited:
- WO-A1-2007/116006
- US-A1- 2005 016 292
- US-A1- 2007 295 501
- US-A1- 2009 198 477
- US-A1- 2010 174 517
- US-B1- 6 561 041
- B Bringendal ET AL: "Application of virtual flow metering as a backup or alternative to multiphase flow measuring devices", , 4 May 2006 (2006-05-04), pages 17-24, XP55005598, [retrieved on 2011-08-25]

## Description

### Field of the Invention

The present invention relates to monitoring the phase composition of production fluid from a hydrocarbon extraction well.

### Background of the Invention

Knowledge of the phase composition of individual fluid production wells within a well complex comprising a production gathering network, enables production optimization strategies to accelerate the production rate of fields. In many instances, these strategies cannot be adopted because of the high cost of having a multiphase flow meter for each well. Previous attempts to overcome this problem are by virtual multiphase flow metering, involving complex optimisation algorithms that are difficult to implement and are sensitive to instrumentation errors. Documents WO2007/116006 A1, US 2010/0174517 A1 and US 2007/0295501 A1 show examples of how to determine the contribution of individual wells in a multiphase production system.

This invention allows the benefits of production optimisation to be gained, without the cost and complexity of having a flow meter for each well, and the method provides a better estimate, as its based on direct measurements.

### Summary of the Invention

According to the present invention from one aspect, there is provided a method of monitoring the phase composition resulting from different phases in production fluid from at least one of the wells of a plurality of subsea hydrocarbon production wells, the method comprising:
modulating the flow of said fluid from said at least one of the wells;
passing the flow of production fluid from all of said wells through a multiphase flow meter common to the wells;
processing data from said multiphase flow meter by means responsive to the modulation of the flow from said at least one of the wells to produce information relating to the phase composition of production fluid from said at least one of the wells, wherein:
   the flow of production fluid from the at least one of the wells is modulated by modulating a respective one of a plurality of chokes through which the production fluid flows from the well by modulating the choke around its nominal set position by a modulating signal from a subsea electronics module of a respective one of a plurality of subsea control modules to provide a relatively small amplitude flow component at a frequency (f) superimposed on the nominal flow for a time period (t).

According to the present invention from another aspect, there is provided a well system comprising a plurality of subsea hydrocarbon production wells, the system including means for monitoring the phase composition resulting from different phases in production fluid from at least one of the wells, the monitoring means comprising:
means for modulating the flow of said fluid from said at least one of the wells;
a multiphase flow meter common to the wells and means for passing the flow of production fluid from all of said wells through said flow meter; and
means for processing data from said multiphase flow meter comprising means responsive to the modulation of the flow from said at least one of the wells to produce information relating to the phase composition of production fluid from said at least one of the wells, wherein:
   the modulating means is adapted for modulating the flow of production fluid from the at least one of the wells by modulating a respective one of a plurality of chokes through which the production fluid flows from the well by modulating the choke around its nominal set position by a modulating signal from a subsea electronics module of a respective one of a plurality of subsea control modules to provide a relatively small amplitude flow component at a frequency (f) superimposed on the nominal flow for a time period (t).

Production fluid from all of said wells could be passed to said flow meter via a manifold common to the wells.

Typically, said different phases comprise oil, gas and water.

Typically, said processing comprises filtering signals from said data from the multiphase flow meter.

The embodiment to be described involves the determination of the phase composition (%oil, %gas, %water) of individual production wells feeding a common manifold, using data from a single downstream multiphase flow meter (MPFM), by modulating, at a frequency of f Hz, the production flow from one well and by digitally filtering the data from the MPFM, at the frequency f, thus extracting the phase composition of that well from the total flow. The process is repeated for each well in turn to reveal the phase composition for all wells feeding the manifold.

### Brief Description of the Drawings

Fig. 1 is a schematic block diagram of part of a well complex in which an embodiment of the invention may be carried out;
Fig. 2 shows typical waveforms resulting from different phases when performing the invention; and
Fig. 3 is a block diagram in respect of the processing of data from an MPFM of Fig. 1.

### Description of an Embodiment of the Invention.

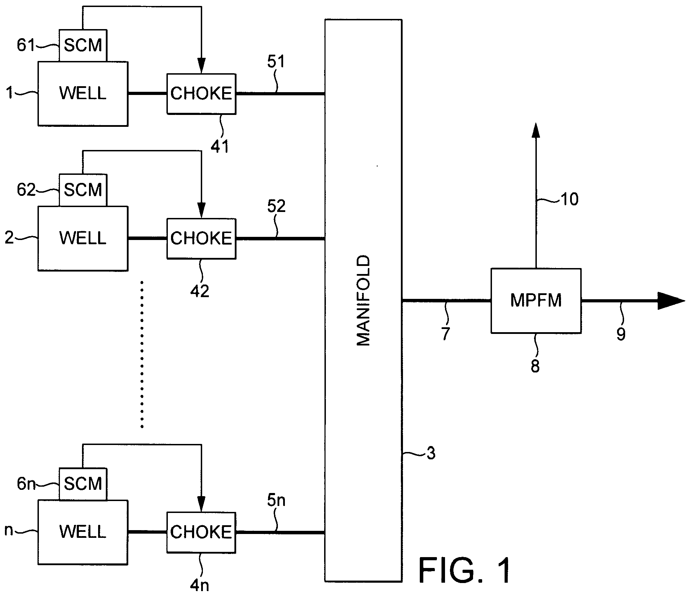

Fig. 1 shows the production flow from a well complex comprising a plurality of subsea hydrocarbon wells 1, 2, ---n, each connected to a common manifold 3. The output of each well passes through a respective one of chokes 41, 42 ---4n to manifold 3 via respective ones of output lines 51, 52 ---5n. Each choke is controlled by a respective one of well subsea control modules (SCMs) 61, 62 ---6n. The combined production flow on a line 7 out of the manifold 3 passes through a single multiphase flow meter (MPFM) 8 common to the wells, before exiting the well complex on an output line 9. The data output on a communication line 10 from the multiphase flowmeter 8 is fed to a topside master control station (MCS) at the surface via the normal communication system of the subsea well complex, where it is processed to provide the phase composition of each of wells 1, 2 ---n.

The mode of operation of the embodiment of the invention is as follows. Starting at the first well 1, its production choke 41 is modulated around its nominal set position by a, typically, sinusoidal modulating signal from the subsea electronics module (SEM) of the SCM 61 to provide a relatively small amplitude flow component at frequency f superimposed on the nominal flow for a time period t. During time t, the downstream MPFM 8 measures the phase composition of the combined flow from all of the wells feeding the manifold 3, including the flow from well 1 and its component modulated at frequency f. During time t, subsea data from the MPFM 8 is transmitted via the normal communication line 10 to the topside MCS, where it is stored. Data relating to production choke 41 of well 1 is also stored and time synchronised with the flow phase data. From the stored data, a time history of the combined production fluid phases can be obtained.

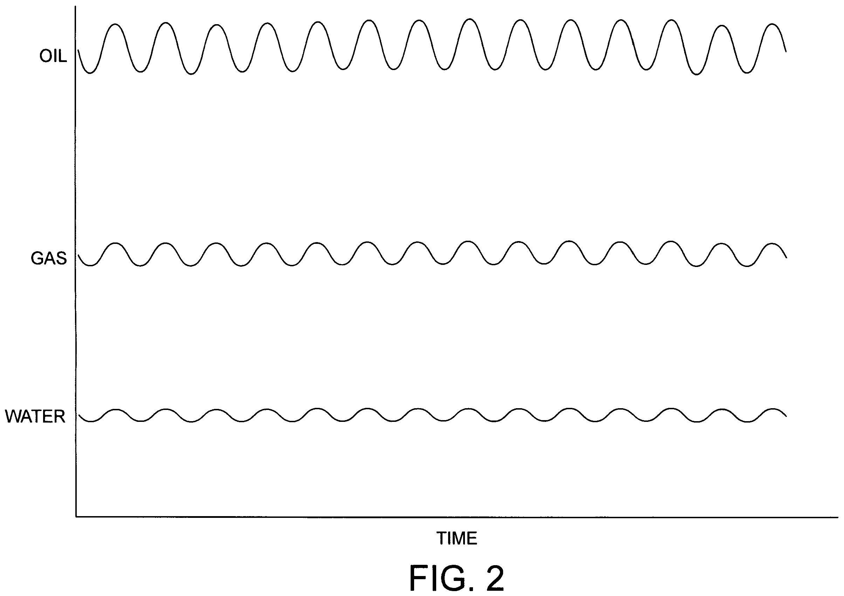

Fig. 2 is a graph that illustrates the typical resultant modulation at frequency f of each of the phases (% oil, % gas, % water) at the output line 51 of the choke 41.

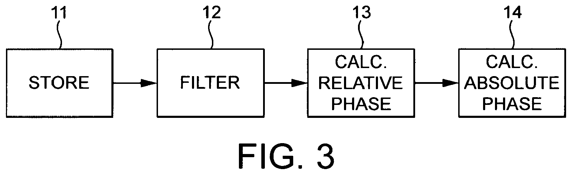

Fig. 3 illustrates the processing of this data stored in a store 11 at the MCS. By using an appropriately designed digital filter 12 and applying this to the time series, the components of the phases modulated at frequency f can be isolated from the combined flow. This provides flow phase data that is specific to well 1 only. The relative phase composition of well 1 (%oil, %gas, %water) can then be determined by a calculator 13 and absolute composition can then be obtained by a calculator 14 by correlating the relative composition against a measure of total flow from well 1, derived from synchronised choke data (position, pressure drop and temperature).

After this process has been completed for well 1, the modulation of well 1 flow will stop and the process repeated for well 2 through to well n such that the phase composition for each well can be determined. The process will then repeat on a cyclic basis.

In practice, the modulation of the chokes 41---4n and thereby the production flows from them is scheduled by the topside MCS.

As an alternative to modulation of the flows from the wells 1---n in a cyclic manner, they could be modulated simultaneously if the modulation frequencies are suitably separated, with demodulation of data from the MPFM8 occurring in the topside MCS.

### Advantages of using the Invention

Multiphase measurements for each well with dramatically reduced number of multiphase flowmeters.

Enablement of production optimisation services.

No additional sensors or complex virtual flow metering algorithms are needed.

## Claims

1. A method of monitoring the phase composition resulting from different phases in production fluid from at least one of the wells of a plurality of subsea hydrocarbon production wells (1, 2 ---n), the method comprising:
modulating the flow of said fluid from said at least one of the wells;
passing the flow of production fluid from all of said wells through a multiphase flow meter (8) common to the wells;
processing data from said multiphase flow meter by means (11-14) responsive to the modulation of the flow from said at least one of the wells to produce information relating to the phase composition of production fluid from said at least one of the wells, wherein:
the flow of production fluid from the at least one of the wells is modulated by modulating a respective one of a plurality of chokes (41, 42 ---4n) through which the production fluid flows from the well by modulating the choke around its nominal set position by a modulating signal from a subsea electronics module of a respective one of a plurality of subsea control modules (61, 62 ---6n) to provide a relatively small amplitude flow component at a frequency (f) superimposed on the nominal flow for a time period (t).

2. A method according to claim 1, wherein the flow of production fluid from all of said wells (1, 2 ---n) is modulated, said processing being carried out on data from the multiphase flow meter (8) to produce information relating to the phase composition of production fluid for each of said wells.

3. A method according to claim 2, wherein the flows of production fluid from said wells (1, 2 ---n) are modulated in turn, so that information relating to the phase composition of production fluid is produced for each of said wells in turn.

4. A method according to any preceding claim, wherein the flow of production fluid from all of said wells (1, 2 ---n) is passed to said via meter (8) via a manifold (3) common to the wells.

5. A method according to any preceding claim, wherein said different phases comprise oil, gas and water.

6. A method according to any preceding claim, wherein said processing comprises filtering signals from said data from the multiphase flow meter (8).

7. A method according to any preceding claim, wherein said processing is carried out topside.

8. A well system comprising a plurality of subsea hydrocarbon production wells (1, 2n), the system including means for monitoring the phase composition resulting from different phases in production fluid from at least one of the wells, the monitoring means comprising:
means for modulating the flow of said fluid from said at least one of the wells;
a multiphase flow meter (8) common to the wells and means (3) for passing the flow of production fluid from all of said wells through said flow meter; and
means (11-14) for processing data from said multiphase flow meter comprising means responsive to the modulation of the flow from said at least one of the wells to produce information relating to the phase composition of production fluid from said at least one of the wells, wherein:
the modulating means is adapted for modulating the flow of production fluid from the at least one of the wells by modulating a respective one of a plurality of chokes (41, 42-4n) through which the production fluid flows from the well by modulating the choke around its nominal set position by a modulating signal from a subsea electronics module of a respective one of a plurality of subsea control modules (61, 62 ---6n) to provide a relatively small amplitude flow component at a frequency (f) superimposed on the nominal flow for a time period (t).

9. A system according to claim 8, adapted for modulating the flow of production fluid from all of said wells (1, 2 ---n), said processing means (11-14) processing data from the multiphase flow meter (8) to produce information relating to the phase composition of production fluid from each of said wells in use of the monitoring means.

10. A system according to claim 9, wherein the modulating means is adapted for modulating the flows of production fluid from said wells (1, 2 ---n) in turn, the processing means (11-14) being adapted for producing information relating to the phase composition of production fluid from each of said wells in turn.

11. A system according to any of claims 8 to 10, wherein said passing means comprises a manifold (3) common to the wells (1, 2 ---n).

12. A system according to any of claims 8 to 11, wherein said processing means (11-14) comprises filtering means (12).

13. A system according to any of claims 8 to 12, wherein said processing means (11-14) is located topside.

## Patentansprüche

1. Verfahren zum Überwachen der Phasenzusammensetzung, die aus unterschiedlichen Phasen in der Produktionsflüssigkeit aus mindestens einem der Bohrlöcher aus einer Vielzahl von unterseeischen Kohlenwasserstoffproduktionsbohrlöchern (1, 2 ---n) resultiert, wobei das Verfahren Folgendes umfasst:
Modulieren des Durchflusses der Flüssigkeit aus dem mindestens einen der Bohrlöcher;
Leiten des Durchflusses von Produktionsflüssigkeit aus allen der Bohrlöcher durch einen Multiphasen-Durchflussmesser (8), der allen Bohrlöchern gemein ist;
Verarbeiten von Daten von dem Multiphasen-Durchflussmesser durch Mittel (11-14) als Reaktion auf die Modulation des Durchflusses aus dem mindestens einen der Bohrlöcher, um Informationen zu erzeugen, die sich auf die Phasenzusammensetzung von Produktionsflüssigkeit aus dem mindestens einen der Bohrlöcher beziehen, wobei:
der Durchfluss von Produktionsflüssigkeit aus dem mindestens einen der Bohrlöcher moduliert wird, indem eine jeweilige aus einer Vielzahl von Drosseln (41, 42 ---4n) moduliert wird, durch welche die Produktionsflüssigkeit aus dem Bohrloch fließt, indem die Drossel um ihre eingestellte Nennposition durch ein Modulationssignal von einem unterseeischen Elektronikmodul eines jeweiligen aus einer Vielzahl von unterseeischen Steuermodulen (61, 62 ---6n) moduliert wird, um eine relativ kleine Amplitudendurchflusskomponente mit einer Frequenz (f) bereitzustellen, die über den Nenndurchsatz für eine Zeitspanne (t) darübergelegt wird.

2. Verfahren nach Anspruch 1, wobei der Durchfluss von Produktionsflüssigkeit aus allen der Bohrlöcher (1, 2 ---n) moduliert wird, wobei die Verarbeitung an Daten von dem Multiphasen-Durchflussmesser (8) ausgeführt wird, um Informationen zu erzeugen, die sich auf die Phasenzusammensetzung von Produktionsflüssigkeit für jedes der Bohrlöcher beziehen.

3. Verfahren nach Anspruch 2, wobei die Durchflüsse von Produktionsflüssigkeit aus den Bohrlöchern (1, 2 ---n) wiederum so moduliert werden, dass Informationen, die sich auf die Phasenzusammensetzung von Produktionsflüssigkeit beziehen, für wiederum jedes der Bohrlöcher erzeugt werden.

4. Verfahren nach einem der vorhergehenden Ansprüche, wobei der Durchfluss von Produktionsflüssigkeit aus allen der Bohrlöcher (1, 2 ---n) durch den Messer (8) über einen Verteiler (3) geleitet wird, der allen Bohrlöchern gemein ist.

5. Verfahren nach einem der vorhergehenden Ansprüche, wobei die unterschiedlichen Phasen Öl, Gas und Wasser umfassen.

6. Verfahren nach einem der vorhergehenden Ansprüche, wobei die Verarbeitung Filtern von Signalen aus den Daten von dem Multiphasen-Durchflussmesser (8) umfasst.

7. Verfahren nach einem der vorhergehenden Ansprüche, wobei die Verarbeitung überseeisch ausgeführt wird.

8. Bohrlochsystem, umfassend eine Vielzahl von unterseeischen Kohlenwasserstoffproduktionsbohrlöchern (1, 2 ---n), wobei das System Mittel zum Überwachen der Phasenzusammensetzung beinhaltet, die aus unterschiedlichen Phasen in der Produktionsflüssigkeit aus mindestens einem der Bohrlöcher resultiert, wobei das Überwachungsmittel Folgendes umfasst:
Mittel zum Modulieren des Durchflusses der Flüssigkeit aus dem mindestens einen der Bohrlöcher;
einen Multiphasen-Durchflussmesser (8) der den Bohrlöchern gemein ist, und Mittel (3) zum Leiten des Durchflusses von Produktionsflüssigkeit aus allen der Bohrlöcher durch den Durchflussmesser; und
Mittel (11-14) zum Verarbeiten von Daten von dem Multiphasen-Durchflussmesser, die Mittel als Reaktion auf die Modulation des Durchflusses aus dem mindestens einen der Bohrlöcher umfassen, um Informationen zu erzeugen, die sich auf die Phasenzusammensetzung von Produktionsflüssigkeit aus dem mindestens einen der Bohrlöcher beziehen, wobei:
das Modulationsmittel ausgelegt ist, um den Durchfluss von Produktionsflüssigkeit aus dem mindestens einen der Bohrlöcher zu modulieren, indem eine jeweilige einer Vielzahl von Drosseln (41, 42 ---4n) moduliert wird, durch welche die Produktionsflüssigkeit aus dem Bohrloch fließt, indem die Drossel um ihre eingestellte Nennposition durch ein Modulationssignal von einem unterseeischen Elektronikmodul eines jeweiligen aus einer Vielzahl von unterseeischen Steuermodulen (61, 62 ---6n) moduliert wird, um eine relativ kleine Amplitudendurchflusskomponente mit einer Frequenz (f) bereitzustellen, die über den Nenndurchsatz für eine Zeitspanne (t) darübergelegt wird.

9. System nach Anspruch 8, das ausgelegt ist, um den Durchfluss von Produktionsflüssigkeit aus allen der Bohrlöcher (1, 2 ---n) zu modulieren, wobei das Verarbeitungsmittel (11-14) Daten von dem Multiphasen-Durchflussmesser (8) verarbeitet, um unter Verwendung des Überwachungsmittels Informationen zu erzeugen, die sich auf die Phasenzusammensetzung von Produktionsflüssigkeit aus jedem der Bohrlöcher beziehen.

10. System nach Anspruch 9, wobei das Modulationsmittel ausgelegt ist, um wiederum die Durchflüsse von Produktionsflüssigkeit aus den Bohrlöchern (1, 2 ---n) zu modulieren, wobei das Verarbeitungsmittel (11-14) ausgelegt ist, um Informationen zu erzeugen, die sich wiederum auf die Phasenzusammensetzung von Produktionsflüssigkeit aus jedem der Bohrlöcher beziehen.

11. System nach einem der Ansprüche 8 bis 10, wobei das Leitungsmittel einen Verteiler (3) umfasst, der den Bohrlöchern (1, 2 ---n) gemein ist.

12. System nach einem der Ansprüche 8 bis 11, wobei das Verarbeitungsmittel (11-14) ein Filtermittel (12) umfasst.

13. System nach einem der Ansprüche 8 bis 12, wobei das Verarbeitungsmittel (11-14) überseeisch angeordnet ist.

## Revendications

1. Procédé de surveillance de la composition de phase issue de différentes phases dans un fluide de production provenant d'au moins l'un des puits d'une pluralité de puits de production d'hydrocarbures sous-marins (1, 2 ---n), le procédé comprenant :
la modulation de l'écoulement dudit fluide provenant dudit au moins l'un des puits ;
le passage de l'écoulement d'un fluide de production provenant de tous lesdits puits à travers un débitmètre multiphase (8) commun aux puits ;
le traitement de données provenant dudit débitmètre multiphase par des moyens (11 à 14) réagissant à la modulation de l'écoulement provenant dudit au moins l'un des puits pour produire des informations concernant la composition de phase du fluide de production provenant dudit au moins l'un des puits, dans lequel :
l'écoulement du fluide de production provenant dudit au moins l'un des puits est modulé par la modulation de l'un respectif d'une pluralité d'étrangleurs (41, 42 ---4n) à travers lequel le fluide de production s'écoule depuis le puits par la modulation de l'étrangleur autour de sa position de réglage nominale par un signal de modulation provenant d'un module électronique sous-marin de l'un respectif d'une pluralité de modules de commande sous-marins (61, 62 ---6n) pour fournir une composante d'écoulement d'amplitude relativement petite à une fréquence (f) superposée sur l'écoulement nominal pendant une période de temps (t).

2. Procédé selon la revendication 1, dans lequel l'écoulement de fluide de production provenant de tous lesdits puits (1, 2, ---n) est modulé, ledit traitement étant effectué sur des données provenant du débitmètre multiphase (8) pour générer des informations concernant la composition de phase de fluide de production pour chacun desdits puits.

3. Procédé selon la revendication 2, dans lequel les écoulements de fluide de production provenant desdits puits (1, 2 ---n) sont modulés à tour de rôle, de sorte que des informations concernant la composition de phase du fluide de production soient produites pour chacun desdits puits à tour de rôle.

4. Procédé selon l'une quelconque des revendications précédentes, dans lequel l'écoulement de fluide de production provenant de tous lesdits puits (1, 2, ---n) traverse ledit débitmètre d'interconnexion (8) via un collecteur (3) commun aux puits.

5. Procédé selon l'une quelconque des revendications précédentes, dans lequel lesdites différentes phases comprennent du pétrole, du gaz et de l'eau.

6. Procédé selon l'une quelconque des revendications précédentes, dans lequel ledit traitement comprend le filtrage de signaux provenant desdites données provenant du débitmètre multiphase (8).

7. Procédé selon l'une quelconque des revendications précédentes, dans lequel ledit traitement est effectué à la surface.

8. Système de puits comprenant une pluralité de puits de production d'hydrocarbures sous-marins (1, 2, ---n), le système comportant des moyens pour surveiller la composition de phase issue de différentes phases dans un fluide de production provenant d'au moins l'un des puits, les moyens de surveillance comprenant :
un moyen pour moduler l'écoulement dudit fluide provenant dudit au moins l'un des puits ;
un débitmètre multiphase (8) commun aux puits et des moyens (3) pour faire passer l'écoulement de fluide de production provenant de tous lesdits puits à travers ledit débitmètre ; et
des moyens (11 à 14) pour traiter des données provenant dudit débitmètre multiphase comprenant des moyens réagissant à la modulation de l'écoulement provenant dudit au moins l'un des puits pour produire des informations concernant la composition de phase d'un fluide de production provenant dudit au moins l'un des puits, dans lequel :
le moyen de modulation est conçu pour moduler l'écoulement d'un fluide de production provenant dudit au moins l'un des puits en modulant l'un respectif d'une pluralité d'étrangleurs (41, 42 ---4n) à travers lequel le fluide de production s'écoule depuis le puits en modulant l'étrangleur autour de sa position de réglage nominale par un signal de modulation provenant d'un module électronique sous-marin de l'un respectif d'une pluralité de modules de commande sous-marins (61, 62, ---6n) pour fournir une composante d'écoulement d'amplitude relativement petite à une fréquence (f) superposée sur l'écoulement nominal pendant une période de temps (t).

9. Système selon la revendication 8, conçu pour moduler l'écoulement d'un fluide de production provenant de tous lesdits puits (1, 2, ---n), lesdits moyens de traitement (11 à 14) traitant des données provenant du débitmètre multiphase (8) pour produire des informations concernant la composition de phase d'un fluide de production provenant de chacun desdits puits lors de l'utilisation des moyens de surveillance.

10. Système selon la revendication 9, dans lequel le moyen de modulation est conçu pour moduler les écoulements de fluide de production provenant desdits puits (1, 2 ---n) à tour de rôle, les moyens de traitement (11 à 14) étant adaptés pour produire des informations concernant la composition de phase d'un fluide de production provenant de chacun desdits puits à tour de rôle.

11. Système selon l'une quelconque des revendications 8 à 10, ledit moyen de passage comprenant un collecteur (3) commun aux puits (1, 2 ---n).

12. Système selon l'une quelconque des revendications 8 à 11, lesdits moyens de traitement (11 à 14) comprenant un moyen de filtrage (12).

13. Système selon l'une quelconque des revendications 8 à 12, lesdits moyens de traitement (11 à 14) se trouvant à la surface.
